# FASCICULE DE BREVET EUROPEEN

(11) **EP 0 532 439 B1**
(45) Date de publication et mention de la délivrance du brevet: **26.04.1995**
(21) Numéro de dépôt: 92420310.2
(22) Date de dépôt: 11.09.1992
(51) Int. Cl.: A61F 2/34, A61F 2/30

(54) **Cotyle pour prothèse de hanche**
Gelenkpfanne für Hüftprothese
Acetabular cup for hip prosthesis

(30) Priorité: 13.09.1991 FR 9111529
(43) Date de publication de la demande: 17.03.1993
(73) Titulaire: IMPACT, F-01800 Charnoz (FR); Collomb, Jean, F-26800 Portes Les Valence (FR); Majou, Claude, F-01800 Meximieux (FR)
(72) Inventeur: Roussouly, Pierre, F-69005 Lyon (FR); Millon, Joseph, F-73490 La Rivoire (FR); Passot, Jean-Paul, F-42530 St Genest Lerpt (FR); Fayard, Jean Philippe, F-42170 St Just St Rambert (FR); Basso, Maurice, F-83400 Hyeres (FR); Augoyard, Marc, F-69005 Lyon (FR); Peyrot, Jacques, F-69005 Lyon (FR); Hulin, Paul Henri, F-89000 Auxerre (FR); Commarmont, Jacques, F-71000 Saint Remy (FR); Charret, Philippe, F-69270 Fontaine sur Saone (FR); Crouzet, Marc, F-69250 Curis au Mont d'Or (FR); Majou, Claude, F-01800 Meximieux (FR); Collomb, Jean, F-26000 Porte les Valence (FR); Courcelles, Philippe, F-69570 Dardilly (FR)
(74) Mandataire: Laurent, Michel

(56) Documents cités:
- EP-A- 0 213 287
- EP-A- 0 337 513
- EP-A- 0 407 332
- FR-A- 2 549 718
- FR-A- 2 595 562
- FR-A- 2 631 542
- FR-A- 2 633 823
- FR-A- 2 634 372
- FR-A- 2 651 995
- FR-A- 2 653 326
- FR-A- 2 668 924
- GB-A- 2 134 360
- US-A- 4 695 282

## Description

L'invention concerne un nouveau type de cotyle pour prothèse de hanche.

De manière connue, une prothèse comprend fondamentalement deux parties, respectivement une tige destinée à être insérée dans le fémur à remplacer ou à renforcer, portant à son extrémité libre une tête sphérique, venant s'articuler dans un cotyle lui-même engagé dans la cavité cotyloïdienne de l'aile illiaque.

Généralement, ce cotyle est réalisé en deux parties distinctes à savoir respectivmeent :
- une cupule dite de "soutien" destinée à être insérée dans la cavité cotyloïdienne de l'aile illiaque,
- et un noyau ou insert dit de "frottement", destiné à s'insérer étroitement dans la cupule de maintien, de coapter le plus possible à celle-ci, et dont la face interne de forme hémisphérique est destinée à recevoir la tête sphérique de la tige de la prothèse.

En pratique, la cupule de soutien rigide est réalisée notamment en métal, tel que par exemple un alliage de titane, alors que le noyau de frottement est le plus généralement réalisé en matière plastique, telle que notamment en polyéthylène haute densité.

Les caractéristiques du préambule de la revendication 1 sont connues du document US-A-4 695 282.

Dans le cadre de l'utilisation de tels cotyles, l'un des problèmes fondamentaux auquel on se heurte concerne l'ancrage ferme et irréversible de la cupule de soutien dans la cavité cotyloïdienne de l'aile illiaque.

De plus en plus, en effet, on essaye de s'affranchir de la pose de ces cupules avec du ciment pour éviter ter d'une part, toute réaction de l'organisme vis à vis de ce dernier, et d'autre part, pour faciliter leur mise en place.

On a ainsi proposé des cupules de soutien dont la face convexe externe présente des fentes d'expansion de longueur croissante, ouvertes sur l'équateur et disposées sur des demi-grands cercles passant par le pôle. De telles cupules dites d'expansion, fixées au moyen notamment de vis ou de têtons expansibles, permettent certes de s'affranchir du ciment, mais ne donnent pas encore entièrement satisfaction sur le plan de leur fixation définitive, et notamment de la repousse osseuse.

L'invention pallie cet inconvénient. Elle propose un cotyle perfectionné du type en question, facile à réaliser et à mettre en place, et dont la structure favorise l'ancrage et la repousse osseuse, augmentant de manière significative le maintien de la cupule dans la cavité cotyloïdienne.

Ce cotyle pour prothèse de hanche, comprend :
- une cupule de soutien destinée à être positionnée dans la cavité cotyloïdienne de l'aile illiaque ;
- un noyau de frottement, en forme générale de coupole, destiné à être inséré dans la cupule de soutien, et à recevoir la tête d'une prothèse de hanche, ledit noyau comportant sur la partie de sa face externe des encoches régulièrement disposées le long de génératrices et une gorge annulaire sécante desdites encoches, destinée à recevoir un clips métallique, l'ensemble encoche - clips étant destiné à coopérer avec la cupule pour assurer son positionnement angulaire et son maintien dans celle-ci ;
- la surface interne de la cupule comportant des bossages, destinés à coopérer avec d'une part les encoches et le clips du noyau ;
   se caractérise :
- en ce que partie de la surface externe de la cupule de soutien comporte des cannelures usinées dans la masse ;
- et en ce que la cupule de soutien (1) comporte une pluralité d'orifices traversants, régulièrement répartis, destinés à permettre sa fixation dans ladite cavité cotyloïdienne.

En d'autres termes, l'invention consiste à ménager sur la périphérie de la surface externe de ladite cupule de soutien, un relief, destiné à favoriser l'ancrage et la repousse osseuse sur la cupule, et en conséquence à optimiser le maintien de ladite cupule dans la cavité cotyloïdienne.

Avantageusement et en pratique :
- Les cannelures se présentent sous la forme de trois rangées d'éléments en reliefs, parallèles entre elles et au plan perpendiculaire à l'axe de révolution de la cupule, et s'étalant sur environ un tiers de la hauteur de celle-ci ;
- la cupule de soutien est réalisée en un alliage de titane ou en acier inoxydable, et le noyau de frottement est réalisé en polyéthylène haute densité.

Selon une autre forme de réalisation de l'invention, le cotyle est muni d'au moins une patte de fixation malléable, prenant naissance au niveau du bord équatorial dudit cotyle, et destinée à permettre d'assurer un point de fixation supplémentaire de celui-ci au niveau de la périphérie osseuse de son point d'implantation.

En outre, dans une variante de cette forme de réalisation, et notamment, lorsque le mauvais état de l'articulation impose la mise en place d'un greffon osseux au niveau de la cavité cotyloïdienne, le cotyle est muni d'un crochet, s'étendant également radialement à partir de son bord équatorial, mais d'un lieu sensiblement diamétralement opposé à celui de la patte. Ce crochet est destiné à prendre appui dans le bord osseux du trou obturateur sous la cavité acétabulaire, afin de s'opposer à tout risque de bascule du greffon.

La manière dont l'invention peut être réalisée et les avantages qui en découlent ressortiront mieux de l'exemple de réalisation qui suit donné à titre indicatif et non limitatif à l'appui des figures annexées.

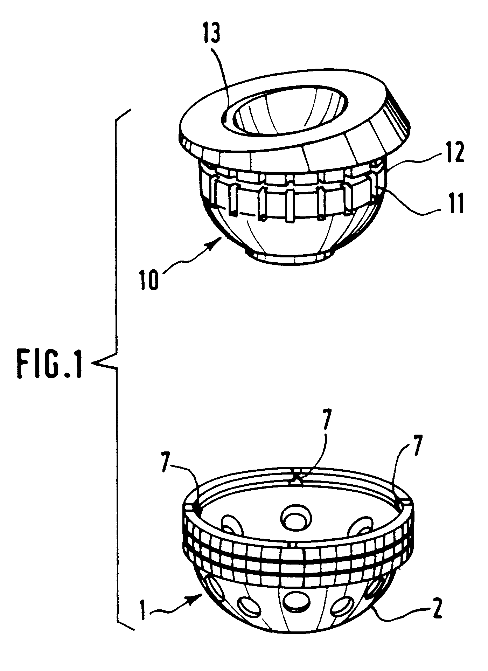

La figure 1 est une vue schématique montrant les deux éléments fondamentaux séparés du cotyle de l'invention.

La figure 2 est une vue schématique en perspective, représentant la cupule de soutien de l'invention.

La figure 3 est une coupe transversale de la cupule de soutien.

La figure 4 est une vue du dessous de la cupule de soutien de l'invention.

La figure 5 est une vue schématique du dessous du cotyle selon une autre forme de réalisation de l'invention.

Les figures 6 et 7 sont des vues schématiques respectivement de profil et de dos du cotyle de la figure 5.

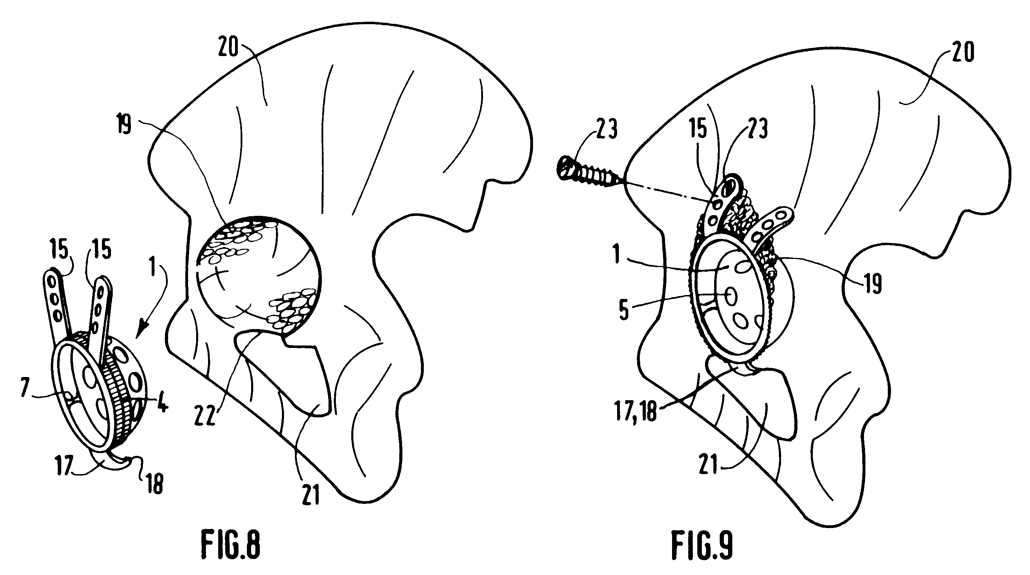

La figure 8 est une représentation schématique d'une aile illiaque et du cotyle selon les figures 5 à 7, préalablement à sa mise en place.

La figure 9 est une vue analogue à la figure 8, le cotyle étant en place dans la cavité acétbulaire.

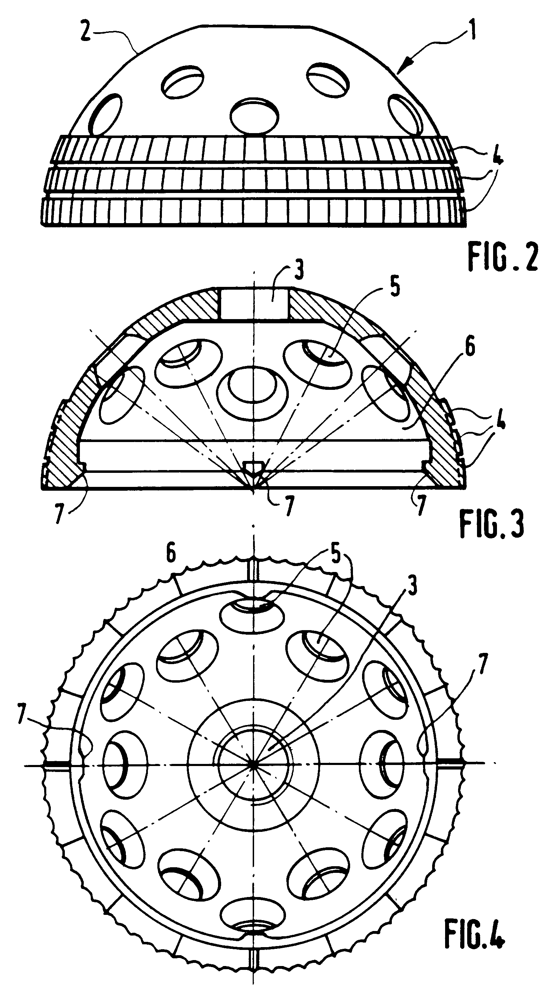

La cupule de soutien conforme à l'invention, désignée sous la référence générale (1), est montrée en détail dans les figures 2 à 4. Elle est réalisée en métal, par exemple en acier inoxydable ou en titane. Elle présente une face externe (2) convexe, hémisphérique, se terminant au voisinage de son pôle par une ouverture au niveau de laquelle est ménagé un orifice (3) fileté, destiné à permettre l'adaptation d'un outillage de pose de cette cupule dans la cavité cotyloïdienne. Cette cupule présente une symétrie de révolution.

Selon la caractéristique fondamentale de l'invention, la surface externe (2) de la cupule comporte sur environ le tiers de sa hauteur, des cannelures (4), usinées dans la masse, et destinées à obtenir une fixation primaire en coopération avec la cavité cotyloïdienne lorsque la cupule est en place dans celle-ci. En outre, ces cannelures sont également destinées à favoriser la repousse osseuse au niveau des aspérités ainsi créées, générant de fait une fixation secondaire. Typiquement, la cupule comporte trois rangées successives de cannelures orientées selon des méridiens de la base au premier tiers de la hauteur. Ces rangées sont parallèles entre elles et au plan perpendiculaire à l'axe de révolution de la cupule. Ces cannelures présentent donc également une symétrie de révolution.

En outre, les deux autres tiers de la cupule comportent des orifices traversants et conformés (5), destinés à permettre le passage de vis de fixation d'une part, et d'autres part, l'escamotage des têtes de vis dans l'épaisseur de la dite cupule.

Il convient en effet que la surface interne (6) de la cupule soit le plus lisse possible pour permettre un positionnement correct du noyau de frottement, comme il sera décrit en détail ultérieurement.

Selon une autre caractéristique de l'invention, la surface interne de la cupule comporte à sa base des bossages (7), destinés à coopérer avec des encoches et un clips métallique ménagé au niveau du noyau de frottement. Ces bossages sont également obtenus par usinage.

De manière avantageuse, la face externe de la cupule est revêtue d'hydroxyapatite sur une épaisseur de 80 à 100 micromètres. Corrélativement, le revêtement intérieur de la cupule peut subir un microbillage, afin de le rendre le plus lisse possible.

Cette cupule (1) est destinée à recevoir un noyau ou insert de frottement (10), typiquement réalisé en polyéthylène haute densité. Cet insert (10) de forme générale hémisphérique, est décrit en détail dans le document FR-A-2 668 924 de l'un des Demandeurs, de sorte qu'il y a lieu de s'y reporter pour sa description détaillée. Comme déjà dit, il comporte sur sa surface externe des encoches (11), et une gorge sécante avec lesdites encoches (11), ladite gorge (12), parallèle au plan perpendiculaire à l'axe de révolution dudit insert, étant destinée à recevoir un clips métallique non représenté, lui-même destiné à coopérer avec les bossages (7) de la cupule. De manière connue, le noyau de frottement (10) est introduit à une température ambiante dans la cupule, et s'expanse à la température du corps humain jusqu'à aboutir à une coaptation maximum entre le noyau et la cupule, et ce afin de diminuer le plus possible le fluage du polyéthylène. En outre, cette coaptation maximum permet de transmettre le plus fidèlement possible les efforts entre la tête prothétique et la cupule. Ce noyau comporte en outre un chanfrein (13) destiné à augmenter le débattement de l'articulation, et qui en outre par son positionnement inférieur, évite un effet de came qui de manière connue provoque la luxation de l'articulation ainsi prothésée. Le nombre d'encoches (11) est avantageusement de seize, espacées régulièrement, afin de permettre un meilleur ajustage du positionnement angulaire du noyau dans la cupule.

La mise en place du cotyle conforme à l'invention s'effectue de la manière suivante. De manière générale, le chirurgien effectue un fraisage sphérique dans la cavité cotyloïdienne, mais d'un diamètre inférieur à celui de la cupule, et notamment des cannelures externes. Il positionne alors en force la cupule de soutien (1) dans cette cavité, en utilisant un outil adapté à l'orifice (3) de ladite cupule. Il procède alors à la fixation de celle-ci par trois ou quatre vis qu'il insère à travers les orifices (5). La cupule de soutien se trouve ainsi positionnée et fixée.

Il introduit alors le noyau de frottement à l'intérieur de la cupule de soutien à la température ambiante, et règle angulairement son orientation par coopération des bossages (7) avec les cannelures (11).

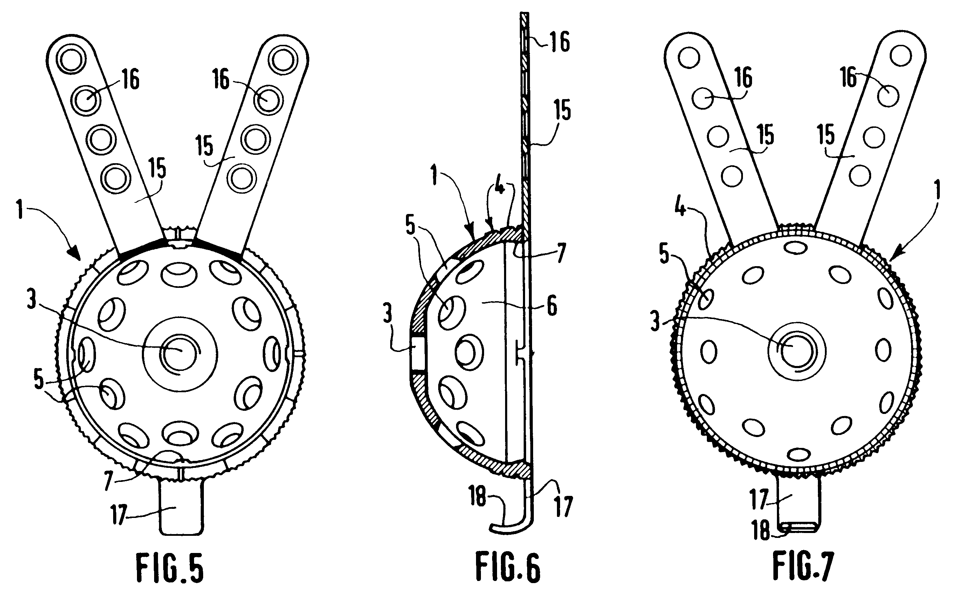

Dans une autre forme de réalisation représentée au sein des figures 5 à 7, le cotyle (1) comporte deux pattes malléables (15), issues de sa base équatoriale, et s'étendant sensiblement radialement par rapport au centre du cotyle en direction de l'extérieur. Ces deux pattes (15) sont multiperforées (16), lesdites perforations (16) étant en outre profilées afin de permettre de recevoir la tête de vis de fixation, lorsque le cotyle est en place au niveau d'une cavité cotyloïdienne. La fixation supplémentaire du cotyle par le biais de cette paire de pattes, dont la malléabilité permet au chirurgien d'adapter leur forme au relief osseux entourant la cavité cotyloïdienne, confère au cotyle une stabilité accrue, de par la contribution desdites pattes (15) à la fixation primaire obtenu par l'appui du bord équatorial dudit cotyle sur la périphérie osseuse.

En outre, ces pattes (15) sont tout particulièrement adaptées lors de la mise en place au niveau de l'aile illiaque (20), préalablement au positionnement du cotyle, de greffons (19), ou d'implants osseux, nécessaires dans le cas d'articulation passablement déteriorée. Les pattes (15) sont alors, de par leur malléabilité, adaptées à la forme des greffons (19), et leur fixation est assurée par une ou plusieurs vis corticales (23), passant par les orifices (16) au travers desdits greffons (19) pour venir se fixer dans la partie acétabulaire ou illiaque de l'articulation.

Lorsqu'un manque de couverture cotyloïdienne apparait, il est courant de réaliser une butée osseuse. La fixation de celle-ci est alors obtenue par lesdites pattes (15), selon le processus décrit ci-dessus.

Enfin, afin d'interdire tout risque de bascule ou d'ascencion de l'implant (19), ou lorsqu'une reconstruction de la cavité acétabulaire s'avère nécessaire, préalablement à la mise en place du cotyle, on prend comme repaire le trou obturateur (21) de l'aile illiaque. De fait, on munit le cotyle (1) d'une patte fixe (17), recourbée (18) en direction du fond du cotyle, en forme de crochet. Cette patte est issue de la base équatoriale du cotyle. Ce crochet vient prendre appui sur le bord osseux (22) dudit trou obturateur (21), et vient, en coopération avec les pattes (15), assurer la stabilité du cotyle. Le crochet (17,18) est diamétralement opposé aux pattes (15).

Ce cotyle pour prothèse de hanche s'avère particulièrement simple de mise en place, dans la mesure où l'on évite le recours au ciment. En outre, son maintien est assuré par une optimisation de l'ancrage et de la repousse osseuse dans lesdites cannelures. On obtient de la sorte une parfaite coaptation de la cupule, grâce à ces reliefs externes.

En outre, compte tenu des différentes pattes de fixation qu'il peut présenter, il s'avère tout particulièrement adapté aux articulations en mauvais état, et procure des résultats que les cotyles connus à ce jour, ne permettaient pas d'obtenir de manière aussi simple et aussi efficace.

## Revendications

1. Cotyle pour prothèse de hanche, du type comprenant :
- une cupule de soutien (1), destinée à être positionnée dans la cavité cotyloïdienne de l'aile illiaque ;
- un noyau de frottement (10), en forme générale de coupole, destiné à être inséré dans la cupule de soutien, et à recevoir la tête d'une prothèse de hanche, ledit noyau comportant sur partie de sa face externe des encoches (11) régulièrement disposées le long de génératrices et une gorge annulaire (12) sécante desdites encoches, destinée à recevoir un clips métallique, l'ensemble encoche - clips étant destiné à coopérer avec la cupule (1) pour assurer son positionnement angulaire et son maintien dans celle-ci ;
- la surface interne de la cupule comportant des bossages (7), destinés à coopérer avec les encoches (11) et le clips du noyau (10) ;
caractérisé :
- en ce que partie de la surface externe (2) de la cupule de soutien (1) comporte des cannelures (4) usinées dans la masse ;
- et en ce que la cupule de soutien (1) comporte une pluralité d'orifices traversants (5), régulièrement répartis, destinés à permettre sa fixation dans ladite cavité cotyloïdienne.

2. Cotyle pour prothèse de hanche selon la revendication 1, caractérisé en ce que les cannelures (4) sont orientées selon des méridiens, et se présentent sous la forme d'au moins trois rangées d'éléments en reliefs, parallèles entre elles et au plan perpendiculaire à l'axe de révolution de la cupule (1), et s'étalant sur environ un tiers de la hauteur de celle-ci .

3. Cotyle pour prothèse de hanche selon l'une des revendications 1 et 2, caractérisé en ce que la cupule de soutien est réalisée en un alliage de titane ou en acier inoxydable.

4. Cotyle pour prothèse de hanche selon l'une des revendications 1 à 3, caractérisé en ce que le noyau de frottement (10) est réalisé en polyéthylène haute densité.

5. Cotyle pour prothèse de hanche selon l'une des revendications 1 à 4, caractérisé en ce qu'il présente au moins une patte mallébale (15), s'étendant radiallement vers l'extérieur à partir de la base équatoriale dudit cotyle, et destinée à venir se fixer sur la périphérie osseuse de la cavité acétabulaire.

6. Cotyle pour prothèse de hanche selon la revendication 5, caractérisé en ce que la patte malléable (15) présente des perforations profilées (16), destinées à permettre le passage de vis de fixation, et à recevoir la tête de celles-ci.

7. Cotyle pour prothèse de hanche selon l'une des revendications 5 et 6, caractérisé en ce qu'il présente un crochet (17,18) dirigé vers le fond du cotyle,s'étendant radiallement vers l'extérieur selon une direction sensiblement diamétralement opposée à celle de la ou des pattes malléables (15), et destiné à coopérer avec le bord osseux (22) du trou obturateur (21) de l'aile illiaque.

## Claims

1. Acetabulum for hip prosthesis, of the type comprising:
- a support cup (1) intended to be positioned in the acetabular cavity of the iliac ala;
- a friction core (10) in the general shape of a dome, intended to be inserted into the support cup and to receive the head of a hip prosthesis, the said core including, over part of its external face, notches (11) regularly arranged along generatrices and an annular groove (12) secant to the said notches, intended to receive a metallic clip, the notch/clip assembly being intended to interact with the cup (1) in order to position it angularly and hold it therein;
- the internal surface of the cup including bosses (7) intended to interact with the notches (11) and the clip of the core (10); characterized :
- in that part of the external surface (2) of the support cup (1) has fluting (4) machined into the mass;
- and in that the support cup (1) includes a plurality of regularly distributed through-orifices (5) intended to allow it to be fastened into the said acetabular cavity.

2. Acetabulum for hip prosthesis according to Claim 1, characterized in that the fluting (4) points along meridians and is in the form of at least three rows of relief elements which are mutually parallel and parallel to the plane perpendicular to the axis of revolution of the cup (1) and spread over approximately one third of the height of the latter.

3. Acetabulum for hip prosthesis according to one of Claims 1 and 2, characterized in that the support cup is made of a titanium alloy or of stainless steel.

4. Acetabulum for hip prosthesis according to one of Claims 1 to 3, characterized in that the friction core (10) is made of high-density polyethylene.

5. Acetabulum for hip prosthesis according to one of Claims 1 to 4, characterized in that it has at least one malleable bracket (15) extending radially outwards from the equatorial base of the said acetabulum and intended to be fastened on the osseous periphery of the acetabular cavity.

6. Acetabulum for hip prosthesis according to Claim 5, characterized in that the malleable bracket (15) has profiled perforations (16) intended to allow passage of fastening screws and to receive the head of the latter.

7. Acetabulum for hip prosthesis according to one of Claims 5 and 6, characterized in that it has a hook (17,18) directed towards the bottom of the acetabulum, extending radially outwards along a direction substantially diametrically opposite that of the malleable bracket or brackets (15) and intended to interact with the osseous edge (22) of the obturator foramen (21) of the iliac ala.

## Patentansprüche

1. Gelenkpfanne für eine Hüftprothese, enthaltend
- eine Stützkuppenschale (1), die dazu vorgesehen ist, in die Vertiefung der Hüftgelenkpfanne des Hüftflügels eingesetzt zu werden;
- eine Reibnuß (10) in der allgemeinen Form einer Kuppel, die dazu vorgesehen ist, um in die Stützkuppenschale eingeführt zu werden und um den Kopf einer Hüftprothese aufzunehmen, wobei die Nuß in einem Bereich auf deren Außenseite Kerben (11), die regelmäßig längs Mantellinien angeordnet sind, und eine die Kerben schneidende Ringnut (12) aufweist, die dazu vorgesehen ist, einen Metallclip aufzunehmen, wobei der Zusammenbau aus Kerben und Clip dazu vorgesehen ist, mit der Kuppenschale (1) derart zusammenzuwirken, daß dessen Winkelstellung und dessen Halterung in dieser sichergestellt ist;
- wobei die Innenseite der Kuppenschale Höcker (7) aufweist, die dazu vorgesehen sind, mit den Kerben (11) und dem Clip der Nuß (10) zusammenzuwirken; dadurch gekennzeichnet,
- daß Bereiche der Außenseite (2) der Stützkuppenschale (1) in das Material eingearbeitete Riffeln (4) aufweisen;
und
- daß die Stützkuppenschale (1) eine Vielzahl an regelmäßig verteilten, durchgehenden Öffnungen (5) aufweist, die dazu vorgesehen sind, deren Befestigung in der Vertiefung in der Hüftgelenkpfanne zu ermöglichen.

2. Gelenkpfanne für eine Hüftprothese nach Anspruch 1, dadurch gekennzeichnet, daß die Riffeln (4) längs Meridianen ausgerichtet sind und in Form von zumindest drei Reliefelementreihen vorliegen, die untereinander parallel verlaufen und in einer Ebene senkrecht zur Drehachse der Kuppenschale (1) liegen, und die sich etwa über ein Drittel der Höhe derselben erstrecken.

3. Gelenkpfanne für eine Hüftprothese nach Anspruch 1 oder 2, dadurch gekennzeichnet, daß die Stützkuppenschale aus einer Titanlegierung oder aus Edelstahl hergestellt ist.

4. Gelenkpfanne für eine Hüftprothese nach einem der Ansprüche 1 bis 3, dadurch gekennzeichnet, daß die Reibnuß (10) aus Polyethylen hoher Dichte herstellt ist.

5. Gelenkpfanne für eine Hüftprothese nach einem der Ansprüche 1 bis 4, dadurch gekennzeichnet, daß zumindest ein sich von der äquatorialen Basis der Gelenkpfanne ausgehendes, radial nach außen erstreckendes biegsames Befestigungselement (15) vorgesehen ist, das dazu bestimmt ist, am Knochenbereich, der die Vertiefung der Hüftgelenkpfanne umrundet, befestigt zu werden.

6. Gelenkpfanne für eine Hüftprothese nach Anspruch 5, dadurch gekennzeichnet, daß das biegsame Befestigungselement (15) profilierte Durchbrüche (16) aufweist, die dazu bestimmt sind, um den Durchtritt von Befestigungsschrauben zu ermöglichen, und um den Kopf dieser aufzunehmen.

7. Gelenkpfanne für eine Hüftprothese nach Anspruch 5 oder 6, dadurch gekennzeichnet, daß sie einen in Richtung des Bodens der Stützkuppenschale gerichteten Haken (17, 18) aufweist, der sich etwa diametral gegenüberliegend zu dem oder den biegsamen Befestigungselementen (15) radial nach außen erstreckt, und der dazu vorgesehen ist, mit dem Knochenrand (22) des Hüftbeinloches (21) des Hüftflügels zusammenzuwirken.
